# EUROPEAN PATENT SPECIFICATION

(11) **EP 1 836 947 B1**
(45) Date of publication and mention of the grant of the patent: **17.06.2009**
(21) Application number: 07005679.1
(22) Date of filing: 20.03.2007
(51) Int. Cl.: A61B 1/005

(54) **Endoscope and display device**
Endoskop und Anzeigevorrichtung
Endoscope et dispositif d'affichage

(30) Priority: 24.03.2006 JP 2006083923
(43) Date of publication of application: 26.09.2007
(73) Proprietor: OLYMPUS MEDICAL SYSTEMS CORP., Tokyo (JP)
(72) Inventor: Watanabe, Katsushi, Tokyo 151-0072 (JP); Shoroji, Ayanori, Tokyo 151-0072 (JP)
(74) Representative: von Hellfeld, Axel

(56) References cited:
- JP-A- 2005 211 204
- US-A- 4 963 960
- US-A1- 2005 070 761

## Description

### BACKGROUND OF THE INVENTION

### 1. Field of the Invention

The present invention relates to an endoscope and, in particular, to an endoscope including a display device integrally and being suitable for mobile use by battery driving.

### 2. Description of the Related Art

Conventionally in the medical fields and industrial fields, an endoscope has been widely spread which is configured to be capable of inserting a long and narrow insert unit into an observation subject to optically observe the inside and perform a treatment thereon by using a treatment instrument inserted into an insertion-channel of the insert unit as required.

For example, in the medical fields, an endoscope is in practical use which includes an insert unit to be inserted into a body cavity to observe the inside of the alimentary canal such as the esophagus, stomach, small intestine and colon and the trachea such as the lungs, for example, and to perform a treatment by using a treatment instrument as required.

Various kinds of electronic endoscopes internally containing an image pickup unit having an image pickup device among the conventional endoscopes have been proposed by Japanese Unexamined Patent Application Publication No. 2005-211204, and so on.

The endoscope disclosed by Japanese Unexamined Patent Application Publication No. 2005-211204 integrally includes a small display device and has a battery such as a small storage battery. The endoscope is configured to be capable of operating by receiving the power supply from the battery. In this configuration, the endoscope by the publication is regarded as being suitable for mobile use.

During an image pickup operation in this endoscope for observing the inside of a body cavity by using the image pickup unit, power is supplied from a power supply device to a light source device, the image pickup unit, a data recording device and an image display device, for example, to perform various kinds of controls for irradiating illumination light, driving an image pickup device, performing a recording operation on a recording medium and displaying image signals. In the endoscope in this case, the irradiation of illumination light by the power supply to the light source device is an operation always required for an image pickup operation.

However, in the conventional endoscope having the configuration as disclosed by Japanese Unexamined Patent Application Publication No. 2005-211204, power is continuously supplied to the light source device during operations other than an image pickup operation, such as a playing operation for displaying an image on an image display device based on recorded image data, and illumination light is continuously irradiated. Thus, the endoscope disadvantageously wastes the power of the battery.

The present invention was made in view of the point, and it is an object of the invention to provide an endoscope which can suppress the exhaustion of power supply means by efficiently supplying power from the power supply means.

### SUMMARY OF THE INVENTION

The above problem can be overcome by an endoscope having the features of claim 1.

An endoscope according to the invention includes a power supply section, a light source device that irradiates illumination light by receiving the supply of power from the power supply section, an image pickup section that picks up a subject image and converts the subject image to an image signal by receiving the supply of power from the power supply section, a recording section that records the image signal by receiving the supply of power from the power supply section, an image display section that receives the input of an image signal from the image pickup section or a signal read out from the recording section and displays an image based on the input signal by receiving the supply of power from the power supply section, a detecting section that detects the input of a signal from the recording section to the image display section, and a control section that controls the power supply to at least one of the light source device and the image pickup section from the power supply section based on the detection result by the detecting section.

The above and other objects, features and advantages of the invention will become more clearly understood from the following description referring to the accompanying drawings.

### BRIEF DESCRIPTION OF THE DRAWINGS

Fig. 1 is an external perspective view showing the entire configuration seen from the back side of an endoscope according to an embodiment of the invention;
Fig. 2 is an external perspective view showing a configuration near an operating unit seen from the front side of the endoscope in Fig. 1; and
Fig. 3 is a block configuration diagram schematically showing an internal configuration of the endoscope in Fig. 1.

### DESCRIPTION OF THE PREFERRED EMBODIMENTS

With reference to the shown embodiment, the invention will be described below.

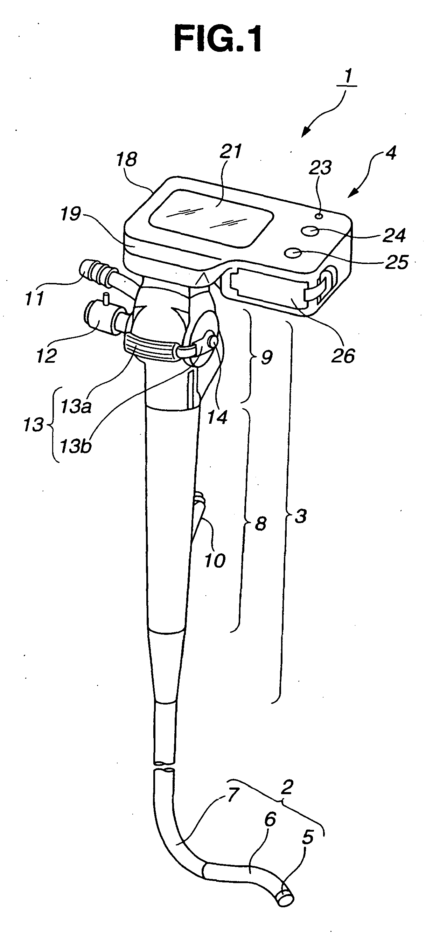

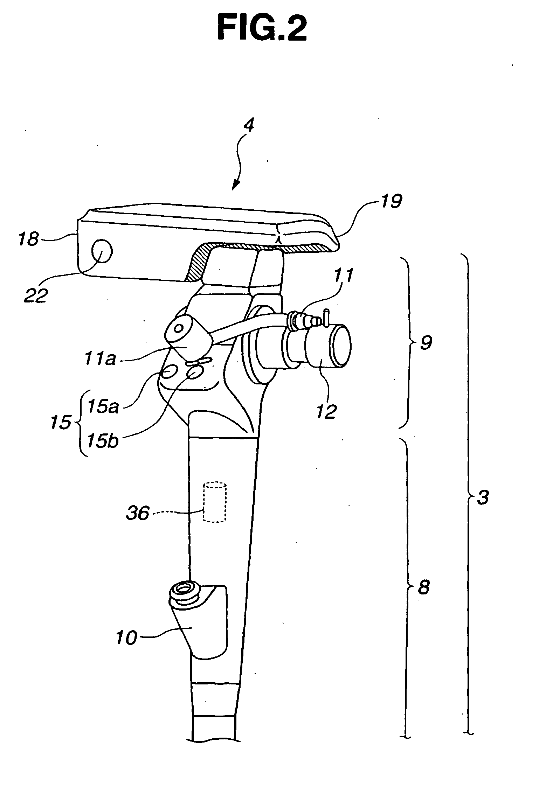

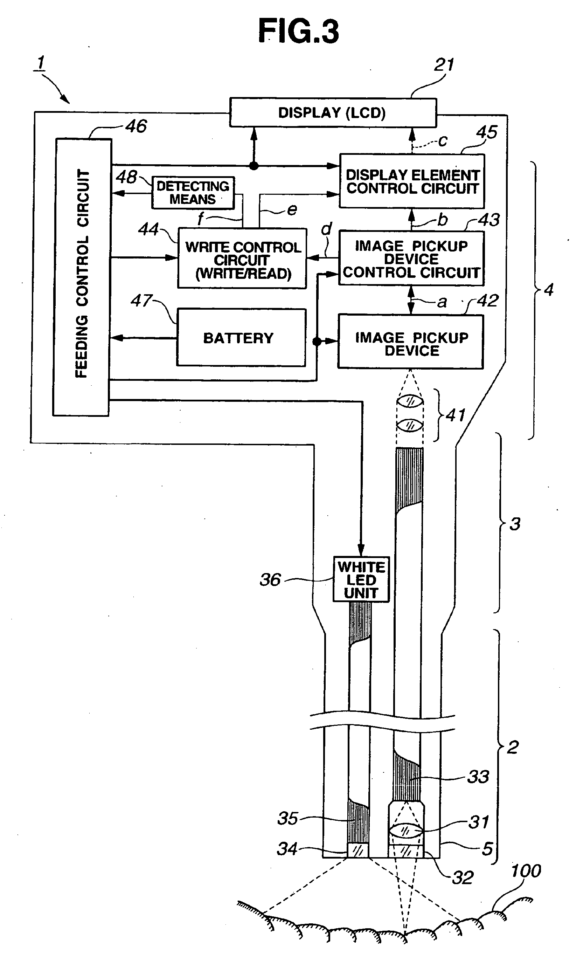

Figs. 1 and 2 are external preservative views showing a configuration of an endoscope of an embodiment of the invention. Fig. 1 of them shows an external view of the whole seen from the back side of the endoscope. Fig. 2 shows an external view near an operating unit seen from the front side of the endoscope. Fig. 3 is a block configuration diagram schematically showing an internal configuration of the endoscope of the present embodiment.

As shown in Fig. 1, an endoscope 1 of the present embodiment mainly includes an insert unit 2, an operating unit 3 and a display device 4. The tip of the insert unit 2 is inserted into a subject part. The operating unit 3 is connected to the base end side of the insert unit 2 and has operating members for performing a bending operation at the tip of the insert unit 2. The display device 4 is provided on the upper end side of the operating unit 3 and displays an endoscopic observation image, for example.

The insert unit 2 has a long and narrow form with flexibility and is connected to the tip side of the operating unit 3. The tip side of the insert unit 2 includes a tip part 5 made of a rigid material, a bendable part 6 connecting to the base end side of the tip part 5, and a flexible part 7 connecting to the bendable part 6 at the tip side and connecting to the tip side of the operating part 3 at the base end side.

The operating unit 3 includes a grasping part 8 to be grasped by a user and an operating unit body 9 at the base end of the grasping part 8.

The grasping part 8 has a stick shape and is gripped by the thumb and the other fingers by a user to grasp the entire endoscope 1. Therefore, the longitudinal direction of the grasping part 8 is defined to be equal to the direction that the fingers excluding the thumb align when a user grips the grasping part 8 by the hand. The grasping part 8 has a forceps insert opening 10 at a middle part thereof for inserting a treatment instrument such as forceps.

The grasping part 8 internally has a white LED unit 36, which is a light source device (see Figs. 2 and 3). The white LED unit 36 functions as a light source device that irradiates illumination light by receiving the supply of power from a power supply means, which will be described later.

The operating unit body 9 includes a suction opening base 11, a vent opening base 12 and a bending lever 13. The suction opening base 11 is for sucking a fluid such as body fluids. The vent opening base 12 is for feeding air into the inside of the endoscope 1 in order to perform a water leak test, for example, on the endoscope 1. The bending lever 13 is for bending the bendable part 6 in a desired direction through an operational wire (not shown) extending through the insert unit 2.

The suction opening base 11 is connectable to a suction system (not shown) through a tube, not shown. The suction opening base 11 extends from the root part of a suction button 11a on the front side of the operating unit body 9 (see Fig. 2). A user may operate the suction system and push the suction button 11a to suck body fluids, for example, through the suction opening base 11.

The vent opening base 12 is connectable to an air feeding system (not shown) through a tube, not shown. A user may operate the air feeding system and feed air from the vent opening base 12 to the inside of the endoscope 1 so that a water leak test can be performed on the inside of the endoscope 1.

The bending lever 13 is placed near the grasping part 8 such that a user can operate it by the thumb of the hand gripping the grasping part 8. The bending lever 13 substantially has an L-shape, and a short arm part 13b is pivotably supported by one side of the operating unit body 9 about a shaft 14.

A long arm part 13a of the bending lever 13 is connected to one end of the short arm part 13b and is placed around the back side (see Fig. 1) of the operating unit body 9. Thus, a user can operate the long arm part 13a of the bending lever 13 by the cushion of the thumb of the hand gripping the grasping part 8.

In this case, the operational direction of the long arm part 13a of the bending lever 13 is equal to the direction along the longitudinal direction of the operating unit 3. In other words, the long arm part 13a of the bending lever 13 is pushed/pulled vertically (that is, in the longitudinal direction of the operating unit 3) by the thumb of the hand to apply tension to one of multiple operational wires extending through the inside of the operating unit 3. Thus, the bendable part 6 can be bent freely in two directions.

Image switches 15 are provided on the front side of the operating unit 3 as shown in Fig. 2. The image switch 15 includes an image recording switch 15a and an image playing switch 15b as shown in Fig. 2. The image recording switch 15a is an operating member for recording an endoscopic observation image, which is captured by the endoscope 1, in a predetermined format by using a write control circuit 44 (which will be described later, see Fig. 3). The image playing switch 15b is an operating member for playing and displaying a recorded endoscopic observation image by using the display device 4.

The display device 4 is integrally connected to the upper side of the operating unit 3. The display device 4 includes a device body 18 substantially having a parallel-piped form and various components on the outer surface of the device body 18.

A tilt adjustable lever (which will be simply called tilt lever) 19 having a plane form extends at one edge of the device body 18. The tilt lever 19 is positioned such that a user can operate it by the thumb of the hand gripping the grasping part 8.

The upper surface of the device body 18 has a display element 21, a power indicator lamp 23, a still image recording select switch 24 and a moving picture recording select switch 25. The display element 21 is image display means for displaying an endoscopic observation image and includes a liquid crystal display (LCD), for example. The power indicator lamp 23 lights up when power is supplied from power supply means (the detail of which will be described later, see Fig. 3) (that is, upon powered-on). The still image recording select switch 24 is used for setting to a still image recording mode for recording a still image, which is a recording mode for an endoscopic observation image. The moving picture recording select switch 25 is used for setting to a moving picture recording mode for recording a moving picture, which is another recording mode for an endoscopic observation image.

A main power switch 22 is provided on one side of the device body 18. The opposite side of the side having the main power switch 22 has a battery 47 (see Fig. 3) and a reclosable lid 26, which is reclosable for attaching/removing a recording medium (not shown) such as a memory card to a predetermined part within the device body 18.

Notably, these components in the device body 18 have a water-tight structure.

With reference to Fig. 3, an internal configuration of the endoscope 1 of the present embodiment will be described next. Fig. 3 shows only components directly relating to the invention, and other components are not shown therein. Therefore, only the components shown in Fig. 3 will be described in detail below, and the components not relating to the invention will not be described since they are the same as those in a conventional general endoscope.

As shown in Fig. 3, in the endoscope 1 of the present embodiment, the front face of the tip part 5 has an observation window 32 and an illumination window 34. The observation window 32 is used for inputting bundles of light forming an image of a subject 100. The illumination window 34 is used for outputting illumination light to the subject 100.

An objective lens 31 for image pickup, which is a part of the image pickup optical system, is provided at the back of the observation window 32 within the tip part 5.

A tip surface of an image guide 33 faces against the back of the objective lens 31. Thus, the bundles of light having entered through the observation window 32 and passed through the objective lens 31 enters from the tip surface of the image guide 33.

The image guide 33 extends through the inside of the insert unit 2 and operating unit 3. The base end surface of the image guide 33 is positioned to face against an image forming lens 41 of the display device 4 connecting to the operating unit 3. Thus, the bundles of light incident on the tip surface of the image guide 33 is output from the base end surface through the image guide 33 and enters the image forming lens 41.

On the other hand, the tip surface of a light guide 35 is provided at the back of the illumination window 34 within the tip part 5.

The light guide 35 extends through the inside of the insert unit 2 and operating unit 3. The base end surface of the light guide 35 is positioned to face against the white LED unit 36 within (the grasping part 8 of) the operating unit 3.

On the other hand, the display device 4 is integrally connected to the upper surface side of the operating unit 3 as described above. The display device 4 mainly includes components including the image forming lens 41, that is, an image pickup device 42, an image pickup device control circuit 43, a write control circuit 44, a display element control circuit 45, the display element 21 such as an LCD, a feeding control circuit 46 and the battery 47, which is a power supply, and detecting means 48, which is a detecting unit.

The image pickup device 42 is positioned to face against the back of the image forming lens 41. The image pickup device 42 is image pickup means in an image pickup unit including an optoelectronic transducer such as a CCD and a CMOS. In other words, the image pickup device 42 captures an optical image of the subject 100 formed by the image forming lens 41, performs optoelectronic transducing processing for transducing it to image signals and generates image signals based on the subject image by receiving the supply of power from the power supply means, which will be described later.

The image pickup device control circuit 43 is a control circuit which may receive image signals generated by the optoelectronic transducing processing by the image pickup device 42, perform various kinds of signal processings and transmit a driving control signal, for example, of the image pickup device 42 to the image pickup device 42. For that reason, the image pickup device control circuit 43 is electrically connected to the image pickup device 42 through a signal line a.

The write control circuit 44 receives image signals from the image pickup device control circuit 43 and creates write image data in an optimum format for recording on a predetermined recording medium (not shown in particular) by receiving the supply of power from the power supply means. The write control circuit 44 may function as recording means forming a recording unit that records the generated data on a self-contained recording medium (not shown), for example, or as playing means forming a playing unit that reads out image data recorded on the recording medium and outputs it to the display element control circuit 45.

For that reason, the write control circuit 44 is electrically connected to the image pickup device control circuit 43 through a signal line d. The write control circuit 44 is further electrically connected to the display element control circuit 45 through a signal line e. The write control circuit 44 is further electrically connected to the feeding control circuit 46 through a signal line f.

The write control circuit 44 includes a recording medium, not shown in particular, that records image data, for example. The recording medium may be a medium in any form such as a removable semiconductor memory in a card shape and a semiconductor memory integrally fixed to the write control circuit 44.

Command signals from the still image recording select switch 24 and the moving picture recording select switch 25, command signals from the image switches 15 (including the image recording switch 15a and image playing switch 15b) in the operating unit 3 are input to the write control circuit 44.

The detecting means 48, which is a detecting unit, detects a signal output from the write control circuit 44 (recording means). As described above, when the image playing switch 15b is operated, the write control circuit 44 in response to the command signal reads out and outputs image data recorded on a recording medium to the display element control circuit 45 (through the signal line e in Fig. 3).

In this case, the detecting means 48 detects a detection signal indicating the fact that image data has been output from the write control circuit 44 by receiving the signal through the signal line f in Fig. 3. The detecting means 48 further detects a command signal generated by the operation on the image recording switch 15a or image playing switch 15b by a user.

The display element control circuit 45 is a control circuit that receives and performs predetermined signal processing on image signals from either image pickup device control circuit 43 or write control circuit 44 and generates display image signals in an optimum format for displaying it on the display element 21 by receiving the supply of power from the power supply means forming the power supply unit. For that reason, the display element control circuit 45 is electrically connected to the display element 21 through the signal line c.

The display element 21 forming the image display unit is image display means that receives display image signals output from the display element control circuit 45 and displays an image in accordance with the received image signals on the display screen. The display element 21 may be a liquid crystal display (LCD), for example, as described above.

The feeding control circuit 46 is a control circuit that controls the power supply to the components within the display device 4 and the white LED unit 36, for example, by receiving the supply of power from the battery 47 including a rechargeable battery, for example. The feeding control circuit 46 may include the main power switch 22, for example.

Therefore, the feeding control circuit 46 receives a command signal from the main power switch 22, for example, or signals from the components and controls the power supply in accordance with the received signal. Here, the feeding control circuit 46 and the battery 47 function as the power supply means.

The feeding control circuit 46 functions as control means forming a control unit that controls the power supply to at least one of the white LED unit 36 (light source device) and the image pickup device 42 (image pickup means) from the power supply means based on the detection result by the detecting means 48.

Having described the case that the detecting means 48 is provided separately from the feeding control circuit 46, the detecting means 48 may be included in the feeding control circuit 46 as an alternative form of this configuration.

In Fig. 3, the thin lines of the solid lines connecting the components indicate the signal lines while thick lines indicate a feeding line for power supply.

In the configuration example in the endoscope 1, the image pickup device 42 (image pickup means) is provided within the display device 4 (image display means), and the white LED unit 36 (light source device) is provided within the operating unit 3, for example. However, the arrangement of both of the components is not limited thereto.

For example, in the endoscope 1, the image pickup means and the light source device may be placed within the tip part 5 of the insert unit 2.

In the endoscope 1, various combinations of arrangements are applicable such as the configuration in which one of the image pickup means and the light source device is placed within the tip part 5 while the other is placed within the operating unit 3 or the display device 4.

Operations of the endoscope 1 of the present embodiment thus configured will be described below.

The endoscope 1 is powered on by a predetermined operation, such as a push operation, on the main power switch 22 by a user. In other words, when a user performs an ON-operation on the main power switch 22, a command signal indicating the ON operation is generated.

In response to the command signal, the feeding control circuit 46 properly controls to supply power from the battery 47 to each of the components of the endoscope 1. Thus, the endoscope 1 starts.

Here, when the endoscope 1 starts, the still image recording mode for recording a still image is normally set. Normally, the still image recording mode continuously displays images captured serially by an image pickup operation by the image pickup device 42 on the display device 21 sequentially and serially as a moving picture. In addition to that, the still image recording mode is an operational mode that allows the current image to be recorded as a still image when a user operates the image recording switch 15a at an arbitrary time.

In other words, when the endoscope 1 starts, the power from the battery 47 to be supplied under the control of the feeding control circuit 46 is supplied to the circuits relating to image pickup, such as the image pickup device 42 and the image pickup device control circuit 43. Thus, an image pickup operation based on the drive of the image pickup device 42, for example, is started.

The image pickup operation to be performed here includes optoelectronic transducing processing by the image pickup device 42 and various kinds of image signal processings by the image pickup device control circuit 43 to be performed by receiving image signals generated by the optoelectronic transducing processing by the image pickup device 42. Here, the image signal processing to be performed by the image pickup device control circuit 43 includes analog-digital conversion processing (A/D conversion processing) for converting image signals (analog signals) from the image pickup device 42 to digital signals and digital image signal processing on the digital image signals generated in this way, for example. Then, the image pickup device control circuit 43 outputs the generated image signals to the display element control circuit 45.

At the same time, power is also supplied from the battery 47 to the circuits relating to image display, such as the display element 21 and the display element control circuit 45. Thus, the display element control circuit 45 receives digital image signals output from the image pickup device control circuit 43 and generates display image signals in an optimum format for display on the display element 21. The display element 21 receives the display image signals from the display element control circuit 45 and displays the image based on the signals on the display screen.

At the same time, power is also supplied from the battery 47 to the circuits relating to data writing, such as the write control circuit 44. Thus, the write control circuit 44 shifts to a standby mode for monitoring command signals from the still image recording select switch 24 and the moving picture recording select switch 25, for example, of the display device 4 and command signals from the image switches 15 (including the image recording switch 15a and image playing switch 15b) in the operating unit 3.

Further at the same time, power is supplied from the battery 47 to the white LED unit 36. Then, the white LED unit 36 starts an operation for emitting illumination light. Thus, the illumination light is irradiated to the front through the light guide 35 and the illumination window 34 on the tip surface of the insert unit 2 of the endoscope 1.

In this way, in the endoscope 1 immediately after it starts, an image pickup operation by the image pickup device 42, for example, an image display operation by the display element 21, for example, and, at the same time, an illumination light irradiating operation by the white LED unit 36 are started.

A user may direct the endoscope 1 in the image-capturable state to a subject part within a body cavity, for example. Thus, the user can perform a desired observation/examination.

Here, the display screen of the display device 4 in the endoscope 1 displays an endoscopic observation image based on an image pickup operation by the image pickup device 42, for example. A user may perform an inserting operation of the endoscope 1 by watching the display screen at the same time. Then, when a user gets to a desired part to record the image, the user may temporarily stop the inserting operation of the endoscope 1 and perform a predetermined operation such as a pushing operation on the image recording switch 15a in the operating unit 3. Then, the write control circuit 44 receives a command signal from the image recording switch 15a and performs a predetermined image recording operation.

In other words, in response to the command signal from the image recording switch 15a, the write control circuit 44 transmits a command signal to perform the image recording operation to the image pickup device control circuit 43 first. In response thereto, the image pickup device control circuit 43 outputs to the write control circuit 44 the image signal captured at the time point when the command signal is received (that is, when the command signal of the image recording switch 15a is generated).

In response thereto, the write control circuit 44 temporarily stores the image signal from the image pickup device control circuit 43 in a self-contained internal memory. Then, the write control circuit 44 performs image signal processing such as signal compression processing, which is optimum for recording on a recording medium, on the image signal and creates image data in a predetermined format. The image data created in this way is recorded on the recording medium connecting to the write control circuit 44.

During the operation for recording the still image data by the write control circuit 44, an image pickup operation and an illumination light irradiating operation are continuously being performed.

Next, when the moving picture recording select switch 25 in the display device 4 is operated by a user while the endoscope 1 is operating in the still image recording mode, the write control circuit 44 receives the command signal generated by the moving picture recording select switch 25 and transmits a predetermined control signal, that is, a control signal for switching the recording mode from the still image recording mode to the moving picture recording mode to the image pickup device control circuit 43.

In response thereto, the image pickup device control circuit 43 switches the driving control over the image pickup device 42 from the driving control in accordance with the still image recording mode to the driving control in accordance with the moving picture recording mode. Thus, the image pickup device 42 starts an operation under the driving control for recording a moving picture.

More specifically, for example, the image pickup device control circuit 43 may sequentially perform signal processing on the image signal received from the image pickup device 42 and then output it to the display element control circuit 45 and, at the same time, also sequentially output it to the write control circuit 44. In response thereto, the write control circuit 44 sequentially and temporarily records the image signal to the self-contained internal memory.

Then, the image pickup device control circuit 43 performs predetermined signal processing on image signals during the period from the time when the command signal from the image recording switch 15a is generated to the time when the command signal from the image recording switch 15a is generated again to create data and records the resulting moving picture data on a recording medium. In the moving picture recording mode, an image pickup operation and illumination light irradiating operation are continuously being performed during the operation for recording moving picture data.

When the still image recording select switch 24 in the display device 4 is operated by a user while the endoscope 1 is operating in the moving picture recording mode, the write control circuit 44 receives the command signal generated by the still image recording select switch 24 and transmits a predetermined control signal, that is, a control signal for switching the recording mode from the moving picture recording mode to the still image recording mode to the image pickup device control circuit 43.

In response thereto, the image pickup device control circuit 43 switches the driving control over the image pickup device 42 from the driving control in accordance with the moving picture recording mode to the driving control in accordance with the still image recording mode. Thus, the image pickup device 42 starts an operation under the driving control for recording still images.

More specifically, for example, the image pickup device control circuit 43 may sequentially perform signal processing on the image signal received from the image pickup device 42 and then output it to the display element control circuit 45 and, at the same time, also wait for a command signal of the image recording switch 15a from the write control circuit 44. When a command signal of the image recording switch 15a is generated, the image pickup device control circuit 43 also outputs the current image signals for one image to the write control circuit 44.

In response thereto, the write control circuit 44 temporarily records the image signals in the self-contained internal memory, performs predetermined signal processing and data conversion on the image signals and records the resulting still image data on a recording medium. In the still image recording mode, an image pickup operation and illumination light irradiating operation are continuously being performed also during the operation for recording still image data.

Having described that an image pickup operation and illumination light irradiating operation are continuously being performed also during the operation for recording still image data in the still image recording mode, at least one of the image pickup operation and the illumination light irradiation operation may be stopped, for example, while the recording operation is being performed for power saving. In other words, in the still image recording mode, when an operation on the image recording switch 15a is detected by the detecting means 48, the detecting means 48 and feeding control circuit 46 may be configured to control the feeding to at least one of the image pickup device 42 and the white LED unit 36 in accordance with the detection result.

In this way, a user may operate arbitrarily either still image recording select switch 24 or moving picture recording select switch 25 at a desired time to switch between the two recording modes, that is, the still image recording mode and the moving picture recording mode in the endoscope 1 and obtain desired image data.

Next, when a user operates the image playing switch 15b when the endoscope 1 is set at the recording mode, that is, at either still image operation mode or moving picture operation mode, the endoscope 1 shifts to the playing mode.

In other words, when a command signal is generated from the image playing switch 15b, the write control circuit 44 in response thereto reads out, decompresses and then outputs the latest image data among image data recorded on the recording medium to the display element control circuit 45. In response thereto, the display element control circuit 45 performs predetermined signal processing to generate image signals in a format suitable for display and outputs the generated image signals to the display element 21. In response thereto, the display element 21 displays the corresponding image.

In this case, when the latest image data recorded on the recording medium is still image data, the display element control circuit 45 performs signal processing for a normal still image. Thus, the display screen of the display element 21 displays the corresponding still image.

On the other hand, when the latest image data on the recording medium is moving picture data, the write control circuit 44 outputs only image signals corresponding to the image data for the first one frame of the moving picture data to the display element control circuit 45. The display element control circuit 45 performs signal processing for a normal still image thereon in response thereto. Thus, the display screen of the display element 21 displays the still image corresponding to the image data for the first one frame of the moving picture data.

Here, the detecting means 48 of the write control circuit 44 detects the fact that the signals have been output from the write control circuit 44 (recording means) to the display element 21 (image display means) through the display element control circuit 45. The detection result by the detecting means 48 is transmitted to the feeding control circuit 46. In response thereto, the feeding control circuit 46 performs power supply control corresponding to the playing mode based on the detection result by the detecting means 48.

In other words, the feeding control circuit 46 performs control (OFF-control) to shut down the power supply from the battery 47 (power supply means) to the image pickup device 42 (image pickup means), image pickup device control circuit 43 and the white LED unit 36 (light source device).

Here, the power supply to the write control circuit 44, display element 21 and display element control circuit 45 is controlled to continue. Thus, the image pickup operation by the image pickup device 42, for example, and the illumination light irradiating operation by the white LED unit 36 temporarily stop.

While one image playing switch (15b) is provided in the present embodiment for the simple switch operation, additional operation switches may be provided in the endoscope 1 for extending playing functions including functions for reading out arbitrary image data and playing/stopping a moving picture in the playing mode. Also in this case, the OFF control as described above is performed.

Next, when a user performs a predetermined operation such as a half-push operation on the image recording switch 15a or performs a push operation on the image playing switch 15b again while the endoscope 1 is operating in the playing mode, the endoscope 1 returns to the recording mode. In this case, the operation mode to return between the still image recording mode and the moving picture recording mode depends on the operation mode set before the operation in the playing mode is performed.

As described above, when a predetermined command signal is generated based on the half-push operation, for example, on the image recording switch 15a while the endoscope 1 is operating in the playing mode, the detecting means 48 of the write control circuit 44 in response thereto detects the breakdown of the output of the signals from the write control circuit 44 (recording means) to the display element control circuit 45. Alternatively, the detecting means 48 detects the start of the input of the image pickup signal from the image pickup device control circuit 43 to the display element control circuit 45 at the same time. In other words, the detecting means 48 detects whether the image signals to be input to the display element control circuit 45 is from the write control circuit 44 or the image pickup device control circuit 43.

The detection result by the detecting means 48 is transmitted to the feeding control circuit 46. In response thereto, the feeding control circuit 46 performs power supply control corresponding to the recording mode based on the detection result by the detecting means 48.

In other words, the feeding control circuit 46 performs control (ON-control) to supply power from the battery 47 to the image pickup device 42 and image pickup device control circuit 43 and the white LED unit 36. Here, the power supply to the write control circuit 44, display element 21 and display element control circuit 45 is controlled to continue. Thus, the image pickup operation by the image pickup device 42, for example, and the illumination light irradiating operation by the white LED unit 36 are both started. By receiving the image signals resulting from the image pickup operation by the image pickup device 42, for example, the image display operation by the display element 21, for example, is also started.

At the same time, the write control circuit 44 shifts to the standby state for monitoring command signals from the still image recording select switch 24, moving picture recording select switch 25, image switches 15 (including image recording switch 15a and image playing switch 15b) and so on.

As described up to this point, according to the present embodiment, the control over the power supply to the components is switched in accordance with the set operation mode in the control over the switching of the operation mode if a user performs an operation for switching the operation mode between the recording mode and the playing mode when the endoscope 1 is available.

Thus, when the endoscope 1 operates in the recording mode, the feeding control circuit 46 controls to supply power to all components in the image pickup, display and illumination systems. On the other hand, when the endoscope 1 operates in the playing mode, the feeding control circuit 46 controls to shut down the power supply to the components in the image pickup and illumination systems, that is, the image pickup device 42 and image pickup device control circuit 43 and the white LED unit 36.

Therefore, the wasteful power consumption can be suppressed by shutting down the power supply to the components which are not required to function when the operation mode is switched from the recording mode to the playing mode. As a result, the exhaustion of the battery 47 can be suppressed.

Since the switching is controlled to supply power to all of required components when the operation mode is switched from the playing mode to the recording mode, a normal image pickup operation can be started immediately only by switching the operation mode without complicated steps.

In the above-described embodiment, the feeding control circuit 46 (control means) performs the power supply control in accordance with the playing mode based on the detection result by the detecting means 48. The power supply control by the feeding control circuit 46 in this case is the control (OFF-control) to shut down the power supply from the battery 47 (power supply means) to the image pickup device 42 (image pickup means) and image pickup device control circuit 43 and the white LED unit 36 (light source device). However, the invention is not limited thereto, but the control may be performed over the power supply from the power supply means to at least one of the white LED unit 36 (light source device) and the image pickup device 42 (image pickup means).

In the above-described embodiment, the write control circuit 44 internally includes the detecting means 48 that detects whether a signal has been output from the write control circuit 44 (recording means) to the display element 21 (image display means) through the display element control circuit 45 or not. Based on the detection result by the detecting means 48, the feeding control circuit 46 controls the power supply to (at least one of) the light source device and the image pickup means from the power supply means.

The means that detects the switching of the operation mode is not limited to the above-described example but may detect the input/output of other signals.

For example, the detecting means 48 may detect a drive-off signal of the image pickup device 42, which is output from the image pickup device control circuit 43, and may transmit the detection result to the feeding control circuit 46. The feeding control circuit 46 in response thereto may control to shut down the power supply to the components such as the image pickup system and the illumination system.

Such means can also control the power supply in the same way as in the embodiment above and can provide the same effects.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

## Claims

1. An endoscope (1) comprising:
power supply means (47);
a light source device (36) that irradiates illumination light by receiving the supply of power from the power supply means (47);
image pickup means (42) for picking up a subject image and converting the subject image to an image signal by receiving the supply of power from the power supply means (47);
recording means (44) for recording the image signal by receiving the supply of power from the power supply means (47);
image display means (21) for receiving the input of an image signal from the image pickup means (42) or a signal read out from the recording means (44) and displaying an image based on the input signal by receiving the supply of power from the power supply means (47), **characterized by**
detecting means (48) for detecting the input of a signal from the recording means to the image display means (21); and
control means (46) for controlling the power supply to at least one of the light source device (36) and the image pickup means (42) from the power supply means (47) based on the detection result by the detecting means (48).

2. The endoscope according to Claim 1, wherein the control means (46) control the power supply to at least one of the light source device (36) and the image pickup means (42) in accordance with a change in the detection signal input from the detecting means (48).

3. The endoscope according to Claim 1 or 2, wherein the control means (46) shuts down the power supply to at least one of the light source device (36) and the image pickup means (42) when a signal is input from the detecting means (48).

4. The endoscope according to Claim 1 or 2, wherein the control means (46) shuts down the power supply to at least one of the light source device (36) and the image pickup means (42) when no signal is input from the detecting means (48).

5. The endoscope according to any one of the preceding claims comprising a display device (4) integrally provided to the endoscope (1), wherein the display device (4) comprises the power supply means (47), the recording means (44), the image display means (21), the detecting means (48) and the control means (46).

## Patentansprüche

1. Endoskop (1) aufweisend:
Enegieversorgungsmittel (47);
eine Lichtquellenvorrichtung (36), welche durch Empfang der Energieversorgung von den Energieversorgungsmitteln (47) Beleuchtungslicht ausstrahlt; Bildaufnahmemittel (42) zum Aufnehmen eines Objektbilds und Umwandeln des Objektbilds in ein Bildsignal durch Empfang der Energieversorgung von den Energieversorgungsmitteln (47);
Aufnahmemittel (44) zum Aufnehmen des Bildsignals durch Empfang der Energieversorgung von den Energieversorgungsmitteln (47);
Bildanzeigemittel (21) zum Empfangen der Zuführung eines Bildsignals von den Bildaufnahmemitteln (42) oder eines aus den Aufnahmemitteln (44) ausgelesenen Signals und Anzeigen eines Bildes basierend auf dem zugeführten Signal durch Empfang der Energieversorgung von den Energieversorgungsmitteln (47),
**gekennzeichnet durch**
Erfassungsmittel (48) zum Erfassen der Zuführung eines Signals von den Aufnahmemitteln an die Bildanzeigemittel (21); und
Steuerungsmittel (46) zum Steuern der Energieversorgung von den Energieversorgungsmitteln (47) zu der Lichtquellenvorrichtung (36) und/oder den Bildaufnahmemitteln (42) basierend auf dem Erfassungsergebnis der Erfassungsmittel (48).

2. Endoskop nach Anspruch 1, bei dem die Steuerungsmittel (46) die Energieversorgung zu der Lichtquellenvorrichtung (36) und/oder den Bildaufnahmemitteln (42) in Übereinstimmung mit einer Änderung des von den Erfassungsmitteln (48) zugeführten Erfassungssignals steuert.

3. Endoskop nach Anspruch 1 oder 2, bei dem die Steuerungsmittel (46) die Energieversorgung zu der Lichtquellenvorrichtung (36) und/oder den Bildaufnahmemitteln (42) abbricht, wenn ein Signal von den Erfassungsmitteln (48) zugeführt wird.

4. Endoskop nach Anspruch 1 oder 2, bei dem die Steuerungsmittel (46) die Energieversorgung zu der Lichtquellenvorrichtung (36) und/oder den Bildaufnahmemitteln (42) abbricht, wenn kein Signal von den Erfassungsmitteln (48) zugeführt wird.

5. Endoskop nach einem der vorhergehenden Ansprüche, aufweisend eine in dem Endoskop (1) integrierte Anzeigevorrichtung (4), wobei die Anzeigevorrichtung (4) die Energieversorgungsmittel (47), die Aufnahmemittel (44), die Bildanzeigemittel (21), die Erfassungsmittel (48) und die Steuerungsmittel (46) aufweist.

## Revendications

1. Endoscope (1) comprenant :
un moyen d'alimentation électrique (47) ;
un dispositif de source de lumière (36) qui projette une lumière d'éclairage en recevant l'alimentation électrique du moyen d'alimentation électrique (47) ;
un moyen de capture d'image (42) pour capturer une image sujet et convertir l'image sujet en un signal d'image en recevant l'alimentation électrique du moyen d'alimentation électrique (47) ;
un moyen d'enregistrement (44) pour enregistrer le signal d'image en recevant l'alimentation électrique du moyen d'alimentation électrique (47) ;
un moyen d'affichage d'image (21) pour recevoir l'entrée d'un signal d'image du moyen de capture d'image (42) ou d'un signal lu dans le moyen d'enregistrement (44) et afficher une image sur la base du signal d'entrée en recevant l'alimentation électrique du moyen d'alimentation électrique (47), **caractérisé par**
un moyen de détection (48) pour détecter l'entrée d'un signal du moyen d'enregistrement dans le moyen d'affichage d'image (21) ; et
un moyen de commande (46) pour commander l'alimentation électrique vers au moins l'un du dispositif de source de lumière (36) et du moyen de capture d'image (42) provenant du moyen d'alimentation électrique (47) sur la base du résultat de détection par le moyen de détection (48).

2. Endoscope selon la revendication 1, dans lequel le moyen de commande (46) commande l'alimentation électrique vers au moins l'un du dispositif de source de lumière (36) et du moyen de capture d'image (42) en fonction d'un changement du signal de détection entré du moyen de détection (48).

3. Endoscope selon la revendication 1 ou 2, dans lequel le moyen de commande (46) coupe l'alimentation électrique vers au moins l'un du dispositif de source de lumière (36) et du moyen de capture d'image (42) lorsqu'un signal est entré du moyen de détection (48).

4. Endoscope selon la revendication 1 ou 2, dans lequel le moyen de commande (46) coupe l'alimentation électrique vers au moins l'un du dispositif de source de lumière (36) et du moyen de capture d'image (42) lorsqu'aucun signal n'est entré du moyen de détection (48).

5. Endoscope selon l'une quelconque des revendications précédentes comprenant un dispositif d'affichage (4) prévu de façon intégrale sur l'endoscope (1), dans lequel le dispositif d'affichage (4) comprend le moyen d'alimentation électrique (47), le moyen d'enregistrement (44), le moyen d'affichage d'image (21), le moyen de détection (48) et le moyen de commande (46).
